# FASCICULE DE BREVET EUROPEEN

(11) **EP 0 729 733 B1**
(45) Date de publication et mention de la délivrance du brevet: **15.01.2003**
(21) Numéro de dépôt: 96400416.2
(22) Date de dépôt: 28.02.1996
(51) Int. Cl.: A61F 5/445, A61F 5/44

(54) **Poche de recueil gonflable notamment pour anus artificiel**
Aufblasbarer Beutel, insbesondere für künstlichen After
Inflatable bag, in particular for artificial anus

(30) Priorité: 01.03.1995 FR 9502359
(43) Date de publication de la demande: 04.09.1996
(73) Titulaire: B. BRAUN BIOTROL, 92107 Boulogne Billancourt (FR)
(72) Inventeur: Holtermann, Henri, 64500 Saint-Jean-de-Luz (FR)
(74) Mandataire: Orès, Bernard

(56) Documents cités:
- EP-A- 0 245 064
- EP-A- 0 248 657
- EP-A- 0 408 296
- WO-A-92/18074
- US-A- 3 902 496
- US-A- 4 319 571
- US-A- 4 710 182

## Description

La présente invention se rapporte à une poche de recueil d'excrétions corporelles et/ou de drainage de plaie, plus particulièrement à une poche pour recueillir les matières s'écoulant d'un anus artificiel.

Dans certaines maladies, il est nécessaire de pratiquer une opération chirurgicale consistant à retirer la partie aval de l'intestin du patient. L'extrémité de la partie de l'intestin laissée en place est alors abouchée à la peau de l'abdomen pour constituer un anus artificiel. Dans ce cas cependant, le patient ne peut plus contrôler l'évacuation des matières. On dispose alors une poche de recueil devant l'anus artificiel pour les recevoir au fur et à mesure de leur écoulement et les stocker.

Les poches les plus utilisées actuellement comportent un patin autoadhésif ayant une ouverture centrale. Ce patin est assujetti à la poche de sorte que son ouverture est en regard d'une ouverture de la poche ménagée dans sa paroi arrière (côté peau). Pour maintenir la poche sur lui, le patient colle à sa peau le patin de façon que l'anus artificiel prend place au sein de l'ouverture du patin. L'anus se trouve ainsi en communication avec l'intérieur de la poche de recueil qui peut dès lors recevoir les matières.

Dans certains modèles de poche dits une pièce, le patin est simplement soudé à la poche. Dans d'autres dits deux pièces, le patin et la poche sont adaptés à être assujettis l'un à l'autre grâce à un raccord qui peut être ouvert et refermé. Cela permet notamment de laisser en place le patin tandis que la poche est retirée pour être changée par exemple.

Quoi qu'il en soit, le port de telles poches peut être douloureux. En effet, elles sont relativement serrées contre l'abdomen par les vêtements et les mouvements du patient ne manquent pas d'engendrer des tiraillements et des pressions au niveau de l'anus artificiel qui se présente souvent comme une plaie ouverte. On a donc songé à munir ces poches de moyens de rembourrage pour en accroître le confort.

Une forme de poche connue est décrite dans la Demande EP 0 408 296. Ses parois sont constituées par un film multicouche de structure comparable à celle des films alvéolaires garnissant l'intérieur d'enveloppes rembourrées. A intervalles réguliers, les couches extérieures du film sont en effet séparées des couches intérieures pour définir entre elles des alvéoles remplies d'air qui font saillie autour de la poche. Par suite, cette dernière ne touche la peau du patient qu'au niveau des alvéoles, ce qui réduit d'autant la surface de contact cutané et la gêne occasionnée.

Une autre forme de poche connue est décrite dans la Demande GB 2 139 501. Sa paroi avant (côté vêtements) et sa paroi arrière (côté peau) sont séparées intérieurement par une paroi intermédiaire imperméable aux liquides, mais perméable aux gaz. Celle-ci évite que les matières recueillies qui pénètrent par l'ouverture de la paroi arrière de la poche, n'entrent en contact avec la paroi avant. Elle autorise en revanche le passage des gaz jusqu'à la paroi avant qui comporte un filtre d'évent. Ce dernier ne risque donc pas d'être encrassé par les matières. Dans un exemple de réalisation, la paroi intermédiaire ne s'étend qu'en partie haute de la poche et son bord inférieur est soudé à la paroi avant de la poche. Avantageusement, une mousse est disposée dans la chambre ainsi créée. La Demande signale que la mousse fait alors office de rembourrage, rendant la poche plus confortable.

L'inconvénient de ces formes de poche connues est que la poche est rembourrée également au niveau de l'anus artificiel. Il s'ensuit que les pressions, notamment celles exercées par les vêtements, portent directement sur l'anus artificiel, même si c'est au travers du rembourrage et qu'elles sont en conséquence quelque peu amorties.

Une autre forme de poche connue est décrite dans le Brevet US 3 902 496 qui pallie cet inconvénient en évitant l'application de pressions directement sur l'anus artificiel. Elle comporte à cette fin deux coussinets s'étendant tous deux verticalement et situés de part et d'autre de l'ouverture de la poche. Les coussinets sont avantageusement formés dans une paroi intermédiaire de la poche qui est repliée sur elle-même et soudée. Ils sont remplis par exemple d'air à la pression atmosphérique.

Un inconvénient de cette dernière forme de poche connue, comme des précédentes d'ailleurs, est que l'épaisseur du rembourrage n'est pas modifiable. Or, la forme de l'anus artificiel peut être très différente selon les patients. Certains, par exemple avec un anus invaginé, désireront une importante épaisseur de rembourrage. D'autres voudront une faible surépaisseur.

Le but de la présente invention est de pallier cet inconvénient grâce à une poche de recueil ayant des moyens de rembourrage dont l'épaisseur peut être adaptée à l'anus artificiel du patient. Cette adaptabilité doit en outre être atteinte sans rendre pour autant les coûts de production de la poche prohibitifs.

Par ailleurs, la Demande PCT WO 92/18074 décrit une poche de recueil qui comprend, outre un patin autoadhésif, un anneau d'étanchéité qui est destiné à entourer étroitement l'anus artificiel de manière à éviter toute fuite de matière entre ce dernier et ledit patin autoadhésif. De ce fait, cet anneau d'étanchéité est rempli d'un fluide dont la pression peut être modifiée à volonté par l'utilisateur pour ajuster le diamètre de l'anneau au diamètre de son anus artificiel et maintenir en toutes circonstances un contact étroit entre eux et, partant, une étanchéité satisfaisante.

La Demande EP 0 248 657 décrit une poche de recueil d'excrétions corporelles liquides du type poche de recueil urinaire, qui est destinée à être reliée au site d'émission de ces excrétions par un cathéter et qui est munie d'une valve anti-retour propre à empêcher que les excrétions qui s'écoulent dans la poche ne refluent vers ce cathéter.

Enfin, la Demande EP 0 245 064 décrit une poche pour l'incontinence fécale qui comprend, entre les deux parois qui la constituent, un coussinet élastique qui est disposé en fer à cheval autour de l'ouverture de la poche et dont la fonction est de maintenir les dites parois suffisamment écartées l'une de l'autre pour obtenir un écoulement satisfaisant des matières fécales dans la poche. L'épaisseur de ce coussinet qui est constitué par un matériau déformable, par exemple une mousse n'est pas modifiable à volonté.

Le problème est résolu selon l'Invention qui propose une poche de recueil notamment d'excrétions corporelles s'écoulant d'un anus artificiel, comportant un réceptacle pour recevoir et stocker les excrétions, ledit réceptacle ayant un pourtour, une paroi avant et une paroi arrière faisant face à la paroi avant et dans laquelle est percée une ouverture pour l'entrée des excrétions, le réceptacle étant adapté pour être maintenu sur un patient avec son ouverture en regard de l'anus artificiel, un rembourrage gonflable et des moyens de gonflage, poche dans laquelle le rembourrage est constitué par un coussinet disposé entre les parois avant et arrière du réceptacle et s'étend depuis le pourtour dudit réceptacle jusqu'à un contour du coussinet situé à une distance de l'ouverture suffisante pour que le bourrelet formé par le coussinet gonflé ne transmette pas de pression à l'anus artificiel lorsque la poche est mise en place.

De la sorte, lé coussinet gonflé aboutit à l'écartement des parois avant et arrière l'une de l'autre. Cela est intéressant afin d'assurer que l'ouverture d'entrée des excrétions ne sera pas obstruée par la paroi avant du réceptacle venant se coller à la paroi arrière. En fait, le coussinet assure un effet de tension sur les parois qui rigidifie la poche et la transforme en une espèce de boîte où les excrétions, même solides, s'écoulent sans entrave. Cela assure également que le coussinet lui-même ne portera pas sur l'anus artificiel. La paroi arrière du réceptacle qui est en regard de la peau immédiatement autour de l'anus n'est ensuite pas serrée par le coussinet contre l'abdomen. Le jeu correspondant peut soulager une zone relativement douloureuse. Mais aussi, la localisation du coussinet le long du pourtour de la poche maximise le volume d'excrétions pouvant être recueillies.

Les moyens de gonflage du coussinet permettent donc au patient de gonfler ce dernier comme il le souhaite. Il est par ailleurs préférable qu'ils autorisent aussi le dégonflage pour le cas où le patient constaterait qu'il a trop gonflé le coussinet.

Avantageusement, les moyens de gonflage comprennent une valve anti-retour ayant au moins une partie à l'intérieur du coussinet. Le patient peut dès lors utiliser une recharge de gaz. Il peut aussi utiliser une seringue pour repousser de l'air dans le- coussinet. Mais il est encore plus commode que la valve ait aussi une partie à l'extérieur adaptée à recevoir un embout permettant à un utilisateur d'y insuffler de l'air en vue de gonfler le coussinet.

Par exemple, la valve est formée par deux parois imperméables aux gaz et souples, se faisant face, soudées l'une à l'autre latéralement et munies de moyens pour rigidifier transversalement les parois de la valve de sorte que ces dernières se collent l'une à l'autre de façon étanche sous l'effet de la pression régnant dans le coussinet. Les moyens pour rigidifier transversalement les parois de la valve sont alors de préférence constitués par une soudure transversale des parois, un passage étant ménagé dans la soudure pour la communication du coussinet avec l'extérieur. Il est en effet aisé de ménager le passage notamment en intercalant une tige métallique entre les deux parois de la valve et par exemple en pressant à chaud la totalité de la largeur des parois en vue de les souder transversalement.

Par exemple encore, la valve est formée par deux pièces rigides qui se terminent du côté de la partie de la valve à l'intérieur du coussinet de façon suffisamment effilée pour qu'elles s'appliquent l'une à l'autre de façon étanche sous l'effet de la pression régnant dans le coussinet. De telles valves sont vendues dans le commerce.

De façon plus pratique, lorsque les parois avant et arrière sont soudées l'une à l'autre le long d'une soudure périphérique qui détermine le pourtour du réceptacle, le coussinet est avantageusement constitué de deux parois imperméables aux gaz et souples, se faisant face et soudées l'une à l'autre au niveau de la soudure périphérique ainsi que le long d'une soudure intérieure suivant le contour du coussinet. Une troisième paroi imperméable aux gaz et souple peut alors avoir ses deux bords pris dans la soudure périphérique du réceptacle de façon à former un soufflet. Ce dernier autorise un plus grand volume de gonflage, mais aussi provoque la mise sous tension des parois avant et arrière de la poche. On réalise ainsi une boîte dans laquelle les excrétions, et plus particulièrement les selles solides, peuvent mieux progresser vers le bas de la poche.

Toujours de façon plus pratique, lorsque les parois avant et arrière sont soudées l'une à l'autre le long d'une soudure périphérique qui détermine le pourtour du réceptacle, le coussinet est avantageusement constitué d'une paroi imperméable aux gaz et souple soudée à la paroi arrière du réceptacle au niveau de la soudure périphérique ainsi que le long d'une soudure intérieure suivant le contour du coussinet. On économise ainsi une paroi pour former le coussinet.

Dans ce cas, la paroi constituant le coussinet peut à la vérité être une paroi intermédiaire du réceptacle, qui est soudée aux parois avant et arrière au niveau de l'intégralité de la soudure périphérique et à la paroi arrière au niveau d'une soudure supplémentaire entourant l'ouverture d'entrée des excrétions de sorte que ces dernières sont exclusivement stockées entre la paroi avant et la paroi intermédiaire. Cette structure permet une fabrication par simple empilement de films qui sont soudés et découpés ensemble sans qu'il soit besoin de se soucier d'un alignement particulier des divers éléments constituant la poche. Elle est beaucoup plus économique. Le cas échéant, la paroi intermédiaire comporte au moins un pli adapté à faire soufflet dans le coussinet pour autoriser un plus grand volume de gonflage et pour assurer la tension des parois de la poche.

Avantageusement, lorsque le réceptacle, une fois mis en place sur le patient, présente une partie haute et une partie basse, le coussinet s'étend au moins en partie haute du réceptacle. En effet, le coussinet gonflé présente une certaine rigidité. Sa présence en haut de la poche empêche la paroi avant de se recourber sous le poids des matières accumulées au fond du réceptacle de la poche. Dans ce cas, il est avantageux de disposer le filtre d'évent sur la paroi avant du réceptacle à la même hauteur que le coussinet, car ce dernier évite alors l'obstruction du filtre par les excrétions ou encore par la paroi arrière du réceptacle.

L'invention sera mieux comprise à la lecture de la description détaillée qui va suivre et à l'examen des dessins annexés qui représentent, à titre d'exemples non limitatifs, des formes de réalisation de l'invention. Sur ces dessins:
- la figure 1 est une vue de face partielle d'une première forme de réalisation d'une poche de recueil selon l'invention. En effet, seule une moitié de poche est montrée, la moitié non dessinée étant la symétrique par rapport à l'axe I-I de la moitié représentée,
- la figure 2 est une coupe selon l'axe de symétrie I-I de la forme de réalisation de la poche de la figure 1. La coupe est par ailleurs représentée en éclaté,
- la figure 3 est une coupe similaire à celle de la figure 2 quant à l'orientation et au mode de représentation en éclaté, mais d'une variante de la forme de réalisation des figures précédentes,
- la figure 4 est une coupe similaire à celles des figures 2 et 3 toujours quant à l'orientation et au mode de représentation en éclaté, mais d'une autre variante de la forme de réalisation des figures précédentes,
- la figure 5 est une vue de face partielle d'une deuxième forme de réalisation d'une poche de recueil selon l'invention. Comme pour la figure 1, seule une moitié de la poche est montrée, la moitié non dessinée étant la symétrique par rapport à l'axe I-I de la moitié représentée,
- la figure 6 est une coupe selon l'axe de symétrie I-I de la forme de réalisation de la poche de la figure 5. La coupe est par ailleurs encore représentée en éclaté,
- la figure 7 est une coupe selon l'axe II-II (cf. figure 5) de la forme de réalisation de la poche de la figure 5. La coupe est par ailleurs toujours représentée en éclaté,
- les figures 8, 9 et 10 sont des vues de face partielles d'autres formes de réalisation de la poche de recueil selon l'invention. Là encore, seule une moitié des poches est montrée, la moitié non dessinée étant la symétrique par rapport à l'axe I-I de la moitié représentée.

Sur les dessins, les éléments des diverses formes de réalisation qui sont équivalents ont reçu des numéros de référence identiques. Par rapport à leur taille réelle, la représentation en éclaté des coupes revient en fait à donner une épaisseur exagérée aux différentes parois des poches de recueil, aux couches de leurs patins et encore plus aux soudures reliant ces éléments. A la vérité, les parois sont des films minces monocouches ou complexes réalisés à partir de matériaux thermoplastiques. Elles ont une épaisseur de l'ordre du dixième de millimètre. Quant aux soudures, réalisées par pression à chaud, hautes fréquences ou ultrasons, elles peuvent aboutir à des épaisseurs encore plus faibles. Elles sont indiquées sur les dessins notamment pour les vues de face par des bandes hachurées. La représentation en éclaté vise en fait à permettre de bien individualiser les différents éléments des poches. Les parois sont enfin supposées transparentes de sorte que des éléments situés derrière la poche sont représentés comme étant visibles. C'est souvent le cas en pratique afin de faciliter la surveillance de leur remplissage en cours d'utilisation.

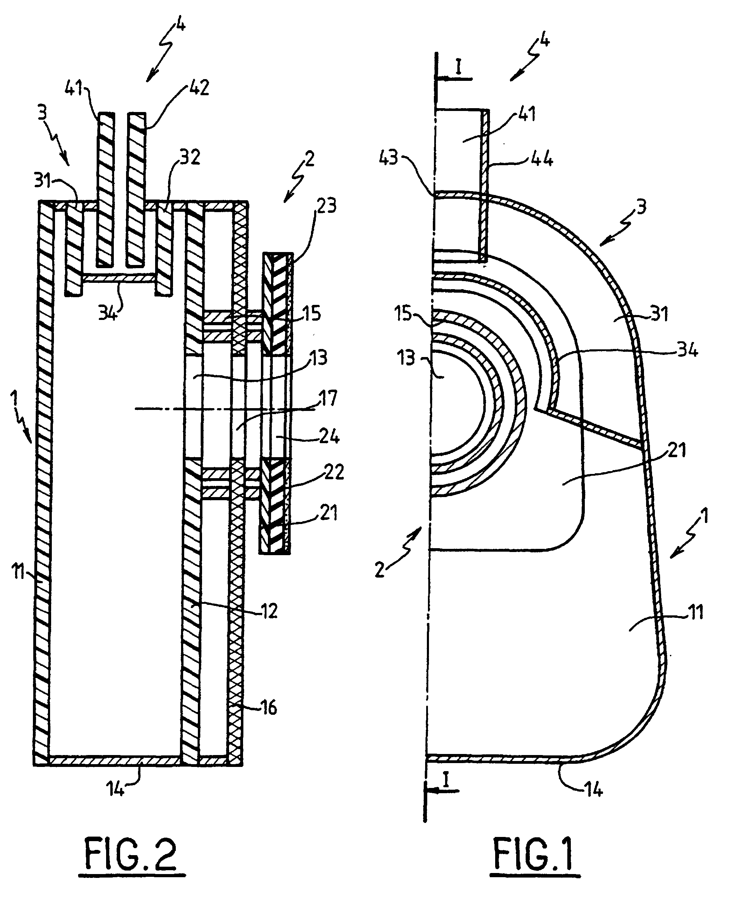

Une première forme de réalisation de la poche de recueil selon l'invention est montrée partiellement en vue de face sur la figure 1 et en coupe éclatée sur la figure 2. Elle est constituée tout d'abord par un réceptacle 1 pour recevoir et stocker les matières. Celui-ci est formé de façon connue par deux parois 11 et 12 qui se font face et sont assujetties de façon étanche l'une à l'autre par une soudure périphérique 14. Sur les dessins, mais sans que cela ne soit impératif dans le cadre de la présente invention, les parois ont une forme trapézoïdale à bords arrondis. La poche se porte de sorte que le plus petit côté du trapèze se trouve en haut. Il y a alors une paroi 11 qui est au contact des vêtements - elle est appelée ici paroi avant - et une paroi 12 qui est au contact de la peau - elle est appelée ici paroi arrière -. A la vérité, un contact direct de cette paroi arrière 12 avec la peau est la plupart du temps évité en raison des risques d'échauffement dus notamment à la transpiration. Un revêtement 16 est assujetti extérieurement à cette paroi arrière 12. Pour cela, il est par exemple soudé avec les parois 11 et 12 au niveau de la soudure périphérique 14. Le revêtement est avantageusement réalisé dans un non tissé.

Dans la moitié supérieure de la paroi arrière 12 est ménagée une ouverture 13 d'entrée des matières. Lorsqu'il y a un revêtement 16, il comporte également une ouverture 17 venant en regard de l'ouverture 13 de la paroi arrière 12. Devant ces ouvertures, à l'extérieur de la poche, est assujetti un patin 2 servant à la fixation de la poche sur le patient. Ce patin 2 comporte lui-même une ouverture 24 qui est alors en regard des ouvertures 13 et 17 et est destinée à recevoir l'anus artificiel. Dans cette forme de réalisation de la poche, le patin 2 est assujetti de façon connue à la fois à la paroi arrière 12 et au revêtement 16 grâce à une ou plusieurs soudures 15 concentriques qui entourent les ouvertures.

Le patin 2 lui-même se compose de façon connue par exemple d'une couche adhésive 22 relativement épaisse (épaisseur de l'ordre du millimètre) appliquée à un substrat 21 et vendue de préférence protégée par une feuille pelable 23. La couche adhésive 22 se compose d'une substance adhésive généralement mélangée à une grande quantité d'hydrocolloïdes. Ceux-ci sont en effet capables d'absorber l'humidité dégagée par la peau, voire les matières. Le patin 2 parvient ainsi à rester collé autour de l'anus artificiel au moins le temps que la poche se remplisse.

De façon spécifique à la présente invention, la poche de recueil comporte en outre un coussinet 3. Dans la forme de réalisation des figures 1 et 2, le coussinet 3 a la forme d'un croissant épousant le bord supérieur de la poche. Il est pour cela formé de deux parois 31 et 32 se faisant face et reliées supérieurement par la soudure périphérique 14 du réceptacle 1 et inférieurement par une nouvelle soudure 34. La matière composant les parois 31 et 32 peut être identique à celle qui compose les parois 11 et 12 du réceptacle. Cela n'est cependant pas une obligation. Il convient simplement qu'il s'agisse d'une matière imperméable aux gaz et souple. Le coussinet 3 est en effet destiné à être gonflé notamment avec de l'air. Dans cet état, les parois 31 et 32 doivent pouvoir se déformer pour donner une sorte de bourrelet.

Lorsque la poche est fixée au patient, ce bourrelet est destiné à prendre place autour de la partie supérieure de l'anus artificiel. Comme, dans la partie inférieure de la poche, des matières ne tardent pas à s'accumuler, l'anus se trouve plus ou moins totalement entouré d'un rembourrage capable d'amortir les pressions transmises en particulier par les vêtements et d'empêcher que ces pressions ne portent directement sur l'anus.

Le coussinet 3 est par ailleurs intérieur au réceptacle. Ainsi, lorsqu'il est gonflé, il aboutit à un écartement des parois avant 11 et arrière 12 l'une de l'autre. Cela a notamment pour avantage d'éviter que le pourtour immédiat de l'ouverture 13 d'entrée des matières ne reste collé à la paroi avant 11, rendant ainsi difficile la pénétration des matières plus avant à l'intérieur du réceptacle 1.

Cela a pour autre avantage que la partie des parois 11 et 12 du réceptacle 1 se trouvant au-dessus de l'ouverture 13 d'entrée des matières ne se recourbe vers l'avant par suite du poids des matières qui s'accumulent au fond du réceptacle 1. Le coussinet 3, sitôt qu'il est gonflé, donne en effet une certaine rigidité à la partie supérieure du réceptacle 1.

De façon également spécifique à la présente invention, une valve de gonflage 4 est prévue pour permettre au patient de gonfler lui-même et comme il l'entend le coussinet 3. Dans la forme de réalisation de la poche des figures 1 et 2, cette valve 4 prend place en partie supérieure de la poche. Elle est formée par deux parois 41 et 42 se faisant face et reliées l'une à l'autre par deux soudures latérales 44. La matière composant les parois 41 et 42 peut être identique à celle qui compose les parois 11 et 12 du réceptacle. Cela n'est cependant pas une obligation. Il convient simplement qu'il s'agisse d'une matière imperméable aux gaz et souple.

En effet, afin de mettre en communication le coussinet 3 avec l'extérieur, les parois 41 et 42 traversent la soudure périphérique 14 du réceptacle 1. A la vérité, elles sont également soudées entre elles le long de cette soudure 14 sauf au niveau d'un mince passage 43 centré par exemple sur l'axe de symétrie I-I. Une façon simple pour obtenir un tel passage 43 consiste, avant la réalisation de la soudure périphérique 14, à disposer entre les parois 41 et 42 une tige métallique de faible diamètre, puis à réaliser la soudure périphérique 14 sur l'intégralité du pourtour du réceptacle 1 comme s'il n'y avait pas de passage 43 à ménager. Après le retrait de la tige, il subsiste le passage 43 voulu tandis que le reste des parois 41 et 42 intéressé par la soudure donne une certaine rigidité à la valve 4.

Cette rigidité est utile pour son fonctionnement comme valve anti-retour. En effet, pour gonfler le coussinet 3, le patient dispose par exemple dans la partie de la valve 4 extérieure au coussinet un embout (non représenté) et souffle dedans. L'air pénètre au travers du passage 43 dans le coussinet 3. Lorsque le patient cesse de souffler, la rigidité de la valve 4 amène les deux parois 41 et 42 à se coller l'une à l'autre sous l'effet de la pression régnant dans le coussinet 3 suite au gonflage. Cela ferme de façon étanche la valve et le coussinet 3 ne se dégonfle pas lorsque le patient retire l'embout de la valve 4.

Le patient peut cependant provoquer le dégonflage. Il lui faut pour cela agir sur la partie de la valve 4 qui est intérieure au coussinet en séparant l'une de l'autre les deux parois 41 et 42 que la pression dans le coussinet maintenait jusque-là collées. Il peut le faire en faisant glisser ces parois l'une contre l'autre au travers des parois 11 et 12 du réceptacle 1. En répétant les opérations de gonflage et de dégonflage du coussinet, le patient parvient à donner au coussinet la forme du bourrelet qu'il souhaite exactement.

La valve 4 pourrait tout aussi bien être constituée d'un clapet rigide (non représenté) fabriqué à part par exemple par moulage. Des clapets formant valve anti-retour sont en effet disponibles dans le commerce. Certains sont par exemple constitués de deux pièces plates, symétriques l'une de l'autre par rapport au plan d'ouverture du clapet et appliquées l'une contre l'autre lorsque le clapet est fermé. Leur section perpendiculaire à ce plan présente à une extrémité la forme d'un biseau effilé. C'est cette extrémité en biseau qui doit alors être insérée dans le coussinet 3. Elle est en effet suffisamment souple pour qu'après le gonflage, la pression régnant dans le coussinet 3 maintienne les deux pièces collées l'une à l'autre comme c'était le cas des parois 41 et 42 de la valve décrite ci-dessus.

L'homme de l'art ne manquera pas de songer à d'autres valves anti-retour qui pourraient être utilisées afin de permettre au patient de gonfler et de dégonfler le coussinet 3 de rembourrage de sa poche de recueil. Il n'est au demeurant pas obligatoire qu'elles soient actionnables en soufflant dedans. D'autres moyens pour introduire l'air tels qu'une seringue font également partie de la présente invention. Le gaz introduit n'est au demeurant pas nécessairement de l'air bien qu'a priori, cela semble le plus commode.

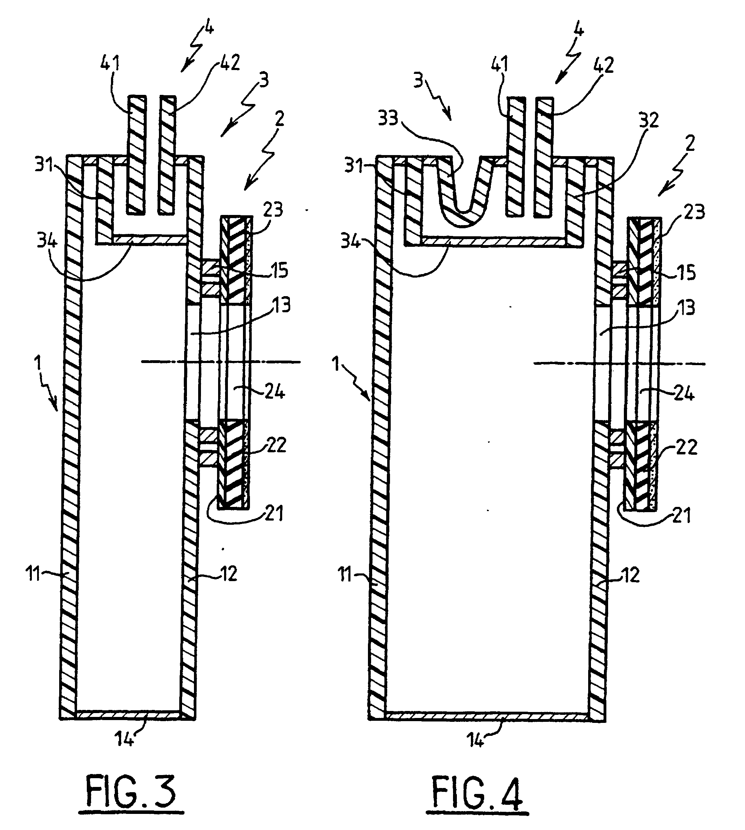

Les poches de recueil représentées sur les figures 3 et suivantes ne diffèrent de la forme de réalisation décrite ci-dessus qu'au niveau de la constitution du coussinet 3 de sorte qu'il ne sera plus question que de cet élément dans les lignes qui suivent.

La variante montrée en coupe sur la figure 3 prévoit une seule paroi 31 pour le coussinet 3. Celle-ci est directement soudée à la paroi arrière 12 du réceptacle 1 le long de sa soudure inférieure 34. Lorsque le coussinet 3 est gonflé, il se forme certes de part et d'autre de cette soudure 34 un sillon relativement inesthétique. Mais, une fois la poche mise en place contre l'abdomen du patient, cela n'est guère visible. En revanche, on économise ainsi la paroi 32, ce qui peut être avantageux en termes de coûts de fabrication.

La variante montrée en coupe sur la figure 4 prévoit un soufflet 33 qui peut être formé dans une bande de matière plastique identique à celle formant les parois 31 et 32 du coussinet et dont les deux bords sont pris dans la soudure périphérique 14. Le volume que peut atteindre, une fois gonflé, le coussinet correspondant est bien plus important que dans les variantes présentées précédemment. Certains patients recherchant un rembourrage particulièrement épais trouveront avantage à de tels coussinets.

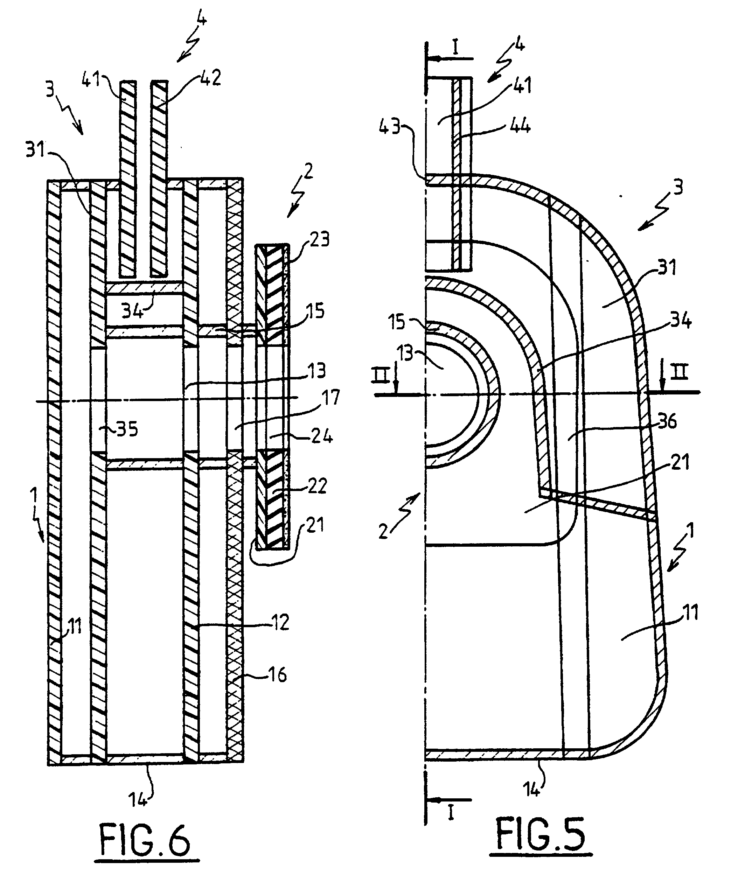

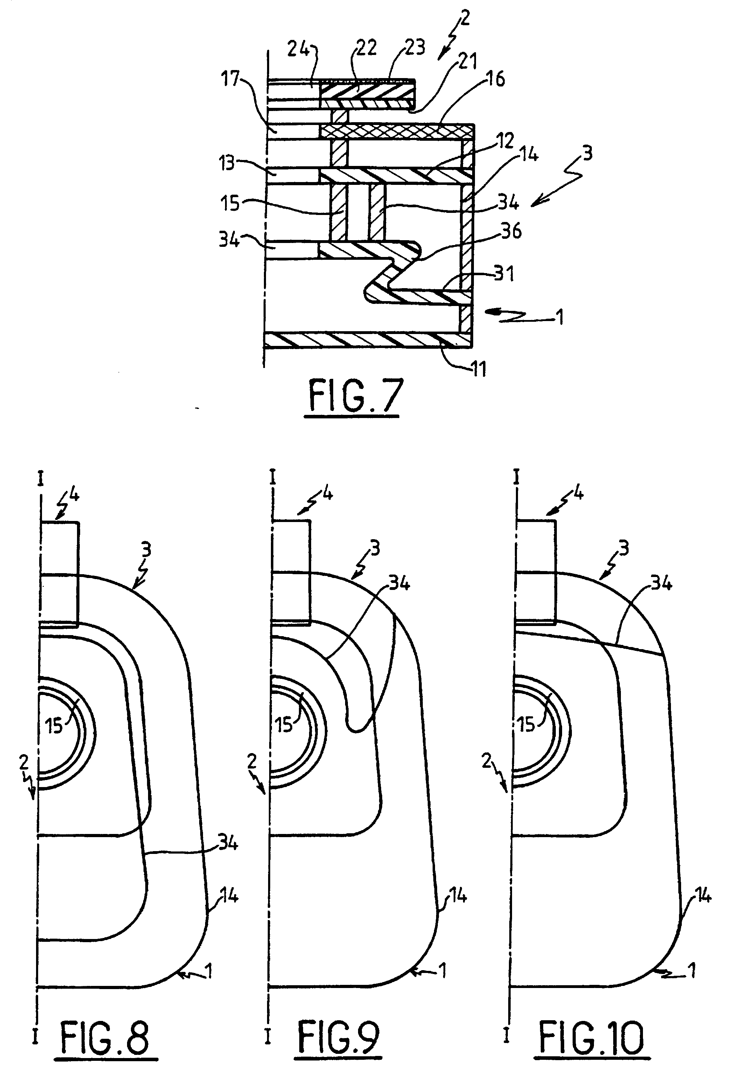

Une deuxième forme de réalisation de la poche de recueil selon l'invention est montrée partiellement en vue de face sur la figure 5 et en coupes éclatées sur les figures 6 et 7. Comme la variante de la figure 3, elle prévoit une seule paroi 31 pour le coussinet 3 qui est directement soudée à la paroi arrière 12 le long de sa soudure 34. Mais, contrairement à cette variante, la paroi 31 intéresse la totalité de la surface du réceptacle 1 de la poche. En d'autres termes, c'est une paroi intermédiaire qui est en outre soudée aux parois avant 11 et arrière 12 au niveau de l'intégralité de la soudure périphérique 14. Pour éviter qu'elle ne détermine au sein du réceptacle deux chambres distinctes, elle est enfin soudée à la paroi arrière 12 ainsi qu'à l'éventuel revêtement 16 le long de la soudure 15 qui entoure les ouvertures 13 et 17 d'entrée des matières. Elle comporte elle-même une ouverture 35 destinée à venir en regard des précédentes de façon que les matières soient stockées entre la paroi avant 11 et la paroi intermédiaire 31.

Une telle paroi 31 intermédiaire est avantageuse essentiellement pour ce qui est de la fabrication de la poche. Il est en effet dès lors possible de
1/ superposer les deux films en matière plastique qui constitueront les parois arrière 12 et intermédiaire 31, puis réaliser la soudure 34 par exemple en pressant à chaud les deux films le long du contour en fer à cheval représenté sur la figure 5,
2/ disposer sur l'empilement d'un patin et d'une feuille de non tissé l'ensemble issu de l'étape 1, puis réaliser la soudure 15 par exemple en pressant à chaud l'empilement complet le long d'un cercle et enfin pratiquer à l'emporte-pièce les ouvertures 24, 17, 13 et 35 respectivement du patin 2, du revêtement 16, de la paroi arrière 12 et de la paroi intermédiaire 31,
3/ disposer sur l'ensemble issu de l'étape 2 un dernier film qui constituera la paroi avant 11, intercaler les parois 41 et 42 préalablement soudées latéralement entre les films qui ont déjà été soudés à l'étape 1 et introduire une tige entre les parois 41 et 42, puis réaliser la soudure périphérique 14 et enfin découper le pourtour du réceptacle avec un emporte-pièce en protégeant la valve 4 dont la partie à l'extérieur du coussinet 3 doit demeurer.

Dans ces étapes, il n'y a pas à se soucier d'aligner précisément des parois autres que celles formant la valve. Or, les dispositifs d'assemblage réalisant de tels alignements sont complexes et coûteux. C'est là un avantage appréciable.

Afin de permettre au coussinet une fois gonflé d'atteindre une épaisseur de rembourrage convenable, la paroi intermédiaire 31 comporte avantageusement deux plis verticaux 36. Sur les coupes partielles des figures 5 et 7, seul l'un d'eux est visible. La coupe selon le plan horizontal II-II permet de bien comprendre comment ce pli fait office de soufflet. Dans l'étape 1 de fabrication présentée ci-dessus, il est par ailleurs aisé de plier longitudinalement le film qui constituera la paroi intermédiaire 31.

Dans les formes de réalisation et variantes qui précèdent, le coussinet 3 est supposé avoir la forme d'un croissant dont le contour inférieur délimité par la soudure 34 s'étend parallèlement au bord supérieur du réceptacle 1 délimité par la soudure périphérique 14. Les figures 8 à 10 montrent d'autres formes possibles pour le coussinet 3. La soudure 34 peut en effet s'étendre parallèlement à la soudure 14 sur la totalité du pourtour du réceptacle comme cela est représenté sur la figure 8.

La soudure 34 peut aussi s'étendre parallèlement à la soudure 14 seulement tout à fait en partie haute du réceptacle comme cela est représenté sur les figures 9 et 10. Sur la figure 9, elle descend ensuite pour s'étendre parallèlement plutôt à la soudure 15 qui entoure l'ouverture d'entrée des matières. Sur la figure 10, elle remonte au contraire de sorte que le coussinet est presque horizontal. Les patients seront amenés à choisir l'une ou l'autre de ces poches en fonction de la forme de leur anus artificiel selon ce qui leur procure le plus de confort.

Le cas échéant, un filtre d'évent (non représenté) est disposé de façon connue sur la face avant 11 du réceptacle 1. Il a alors tout intérêt a être placé devant la paroi 31 du coussinet 3. En cet endroit en effet, les risques d'encrassement par les matières ou encore d'obstruction par la paroi arrière 12 sont moindres. Le bourrelet formé à l'intérieur du réceptacle par le coussinet tend en effet à repousser le haut de la paroi avant.

## Revendications

1. Poche de recueil notamment d'excrétions corporelles s'écoulant d'un anus artificiel, comportant un réceptacle (1) pour recevoir et stocker les excrétions, ledit réceptacle ayant un pourtour (14), une paroi avant (11) et une paroi arrière (12) faisant face à la paroi avant et dans laquelle est percée une ouverture (13) pour l'entrée des excrétions, le réceptacle étant adapté pour être maintenu sur un patient avec son ouverture en regard de l'anus artificiel, un rembourrage gonflable et des moyens de gonflage, **caractérisé en ce que** le rembourrage est constitué par un coussinet (3) disposé entre les parois avant (11) et arrière (12) du réceptacle et s'étend depuis le pourtour (14) dudit réceptacle jusqu'à un contour du coussinet situé à une distance de l'ouverture (13) suffisante pour que le bourrelet formé par le coussinet gonflé ne transmette pas de pression à l'anus artificiel lorsque la poche est mise en place.

2. Poche selon la revendication 1, **caractérisée en ce que** les moyens de gonflage autorisent aussi le dégonflage.

3. Poche selon la revendication 2, **caractérisée en ce que** les moyens de gonflage comprennent une valve anti-retour (4) ayant au moins une partie à l'intérieur du coussinet (3).

4. Poche selon la revendication 3, **caractérisée en ce que** la valve (4) a aussi une partie à l'extérieur adaptée à recevoir un embout permettant à un utilisateur d'y insuffler de l'air en vue de gonfler le coussinet (3).

5. Poche selon la revendication 4, **caractérisée en ce que** la valve (4) est formée par deux parois (41,42) imperméables aux gaz et souples, se faisant face, soudées l'une à l'autre latéralement et munies de moyens pour rigidifier transversalement les parois (41,42) de la valve (4) de sorte que ces dernières se collent l'une à l'autre de façon étanche sous l'effet de la pression régnant dans le coussinet (3).

6. Poche selon la revendication 5, **caractérisée en ce que** les moyens pour rigidifier transversalement les parois (41,42) de la valve (4) sont constitués par une soudure transversale (14) des parois (41,42) , un passage (43) étant ménagé dans la soudure (14) pour la communication du coussinet (3) avec l'extérieur.

7. Poche selon la revendication 4, **caractérisée en ce que** la valve (4) est formée par deux pièces rigides qui se terminent du côté de la partie de la valve à l'intérieur du coussinet (3) de façon suffisamment effilée pour qu'elles s'appliquent l'une à l'autre de façon étanche sous l'effet de la pression régnant dans le coussinet (3).

8. Poche selon la revendication 7, les parois avant (11) et arrière (12) étant soudées l'une à l'autre le long d'une soudure périphérique (14) qui détermine le pourtour du réceptacle (1), **caractérisée en ce que** le coussinet (3) est constitué de deux parois (31,32) imperméables aux gaz et souples, se faisant face et soudées l'une à l'autre au niveau de la soudure périphérique (14) ainsi que le long d'une soudure intérieure (34) suivant le contour du coussinet (3).

9. Poche selon la revendication 8, **caractérisée en ce qu'**une troisième paroi (33) imperméable aux gaz et souple a ses deux bords pris dans la soudure périphérique (14) du réceptacle (1) de façon à former un soufflet autorisant un plus grand volume de gonflage.

10. Poche selon la revendication 7, les parois avant (11) et arrière (12) étant soudées l'une à l'autre le long d'une soudure périphérique (14) qui détermine le pourtour du réceptacle (1), **caractérisée en ce que** le coussinet (3) est constitué d'une paroi (31) imperméable aux gaz et souple soudée à la paroi arrière (12) du réceptacle (1) au niveau de la soudure périphérique (14) ainsi que le long d'une soudure intérieure (34) suivant le contour du coussinet (3).

11. Poche selon la revendication 10, **caractérisée en ce que** la paroi (31) constituant le coussinet (3) est une paroi intermédiaire du réceptacle, qui est soudée aux parois avant (11) et arrière (12) au niveau de l'intégralité de la soudure périphérique (14) et à la paroi arrière (12) au niveau d'une soudure supplémentaire (15) entourant l'ouverture (13) d'entrée des excrétions de sorte que ces dernières sont exclusivement stockées entre la paroi avant (11) et la paroi intermédiaire.

12. Poche selon la revendication 11, **caractérisée en ce que** la paroi intermédiaire comporte au moins un pli (36) adapté à faire soufflet dans le coussinet (3) pour autoriser un plus grand volume de gonflage.

13. Poche selon l'une quelconque des revendications 7 à 12, le réceptacle (1), une fois mis en place sur le patient, présentant une partie haute et une partie basse, **caractérisée en ce que** le coussinet (3) s'étend au moins en partie haute du réceptacle (1).

14. Poche selon la revendication 13, caractérisée en qu'un filtre d'évent est disposé sur la paroi avant (11) du réceptacle (1) à la même hauteur que le coussinet (3).

## Patentansprüche

1. Beutel für die Aufnahme insbesondere von Körperausscheidungen, die einem künstlichen Anus entströmen, umfassend einen Behälter (1) zum Aufnehmen und Speichern der Ausscheidungen, wobei der Behälter einen Umfang (14), eine Vorderwand (11) und eine Rückwand (12), die der Vorderwand gegenüberliegt und in welcher eine Öffnung (13) für den Eintritt von Ausscheidungen angelegt ist, hat, wobei der Behälter dazu geeignet bzw. angepasst ist, auf bzw. an einem Patienten mit seiner Öffnung gegenüber dem künstlichen Anus gehalten bzw. aufrecht erhalten zu werden, sowie eine aufblasbare Polsterung und Mittel für das Aufblasen, **dadurch gekennzeichnet, dass** die Polsterung von einem Auflager (3) gebildet ist, die zwischen der Vorderwand (11) und der Rückwand (12) des Behälters angeordnet ist und sich von dem Umfang (14) des Behälters aus bis zu einer Kontur des Auflagers erstreckt, die sich in einem Abstand von der Öffnung (13) befindet, der genügend ist, dass die von dem aufgeblasenen Auflager gebildete Wulst keinen Druck auf den künstlichen Anus überträgt, wenn der Beutel angebracht bzw. angesetzt ist.

2. Beutel gemäß Anspruch 1, **dadurch gekennzeichnet, dass** die Mittel für das Aufblasen außerdem das Abblasen erlauben.

3. Beutel gemäß Anspruch 2, **dadurch gekennzeichnet, dass** die Mittel für das Aufblasen ein Rückschlagventil (4) umfassen, das wenigstens einen Teil im Inneren des Auflagers (3) hat.

4. Beutel gemäß Anspruch 3, **dadurch gekennzeichnet, dass** das Ventil (4) außerdem einen Teil im Äußeren hat, der dazu geeignet bzw. angepasst ist, ein Ansatzstück aufzunehmen, das es einem Benutzer gestattet, darein Luft zum Aufblasen des Auflagers (3) einzublasen.

5. Beutel gemäß Anspruch 4, **dadurch gekennzeichnet, dass** das Ventil (4) von zwei Wänden (41, 42) gebildet ist, die für Gas undurchlässig und elastisch sind, sowie gegenüberliegen, seitlich aneinandergeschweißt sind und mit Mitteln zur Querversteifung der Wände (41, 42) des Ventils (4) derart ausgerüstet sind, dass die letzteren in dichter Art und Weise unter der Wirkung des Drucks, der in dem Auflager (3) herrscht, aneinander haften.

6. Beutel gemäß Anspruch 5, **dadurch gekennzeichnet, dass** die Mittel zum Querversteifen der Wände (41, 42) des Ventils (4) von einer Querverschweißung (14) der Wände (41, 42) gebildet sind, wobei für die Verbindung des Auflagers (3) mit dem Äußeren ein Durchgang (43) in der Verschweißung (14) angeordnet bzw. ausgespart ist.

7. Beutel gemäß Anspruch 4, **dadurch gekennzeichnet, dass** das Ventil (4) von zwei steifen Teilen gebildet ist, die auf der Seite des Teils bzw. neben dem Teil des Ventils im Inneren des Auflagers (3) in genügend verjüngter Art und Weise enden, damit sie sich unter der Wirkung des in dem Auflager (3) herrschenden Drucks in dichter Art und Weise aneinanderlegen.

8. Beutel gemäß Anspruch 7, wobei die Vorderwand (11) und die Rückwand (12) miteinander längs einer Umfangsverschweißung (14), welche den Umfang des Behälters (1) bestimmt, verschweißt sind, **dadurch gekennzeichnet, dass** das Auflager (3) von zwei für Gas undurchlässigen und elastischen Wänden (31, 32) gebildet ist, die einander gegenüberliegen und miteinander auf dem Niveau der Umfangsverschweißung (14) wie auch längs einer Innenverschweißung (34) gemäß der Kontur des Auflagers (3) verschweißt sind.

9. Beutel gemäß Anspruch 8, **dadurch gekennzeichnet, dass** eine dritte Wand (33), die für Gas undurchlässig und elastisch ist, mit ihren beiden Rändern in der Art und Weise in die Umfangsverschweißung (14) des Behälters (1) genommen ist, dass ein Balg gebildet wird, der ein größeres Aufblasvolumen erlaubt.

10. Beutel gemäß Anspruch 7, wobei die Vorderwand (11) und die Rückwand (12) längs einer Umfangsverschweißung (14), welche den Umfang des Behälters (1) bestimmt, miteinander verschweißt sind, **dadurch gekennzeichnet, dass** das Auflager (3) von einer für Gas undurchlässigen und elastischen Wand (31) gebildet ist, die mit der Rückwand (12) des Behälters (1) auf dem Niveau der Umfangsverschweißung (14) wie auch längs einer Innenverschweißung (34) gemäß der Kontur des Auflagers (3) verschweißt ist.

11. Beutel gemäß Anspruch 10, **dadurch gekennzeichnet, dass** die das Auflager (3) bildende Wand (31) eine Zwischenwand des Behälters ist, welche mit der Vorderwand (11) und der Rückwand (12) auf dem Niveau der Integralität der Umfangsverschweißung (14) und mit der Rückwand (12) auf dem Niveau einer zusätzlichen Verschweißung (15), welche die Öffnung (13) des Eintritts der Ausscheidungen umgibt bzw. umschließt, verschweißt ist, derart, dass diese letzteren ausschließlich zwischen der Vorderwand (11) und der Zwischenwand gespeichert werden.

12. Beutel gemäß Anspruch 11, **dadurch gekennzeichnet, dass** die Zwischenwand wenigstens eine Falte (36) hat, die dazu geeignet bzw. angepasst ist, in dem Auflager (3) einen Balg zu bilden, um ein größeres Aufblasvolumen zu erlauben.

13. Beutel gemäß irgendeinem der Ansprüche 7 bis 12, wobei der Behälter (1), wenn er einmal auf bzw. an dem Patienten an Ort und Stelle gebracht ist, einen oberen Teil und einen unteren Teil aufweist, **dadurch gekennzeichnet, dass** sich das Auflager (3) wenigstens im oberen Teil des Behälters (1) erstreckt.

14. Beutel gemäß Anspruch 13, **dadurch gekennzeichnet, dass** ein Luftabzugsfilter auf der Vorderwand (11) des Behälters (1) auf der gleichen Höhe wie das Auflager (3) angeordnet ist.

## Claims

1. A collecting bag, in particular for collecting body excreta running from an artificial anus, the bag including a receptacle (1) for receiving and storing excreta, said receptacle having a periphery (14), a front wall (11), and a back wall (12) overlying the front wall and in which an inlet opening (13) for excreta is formed, the receptacle being adapted to be held on a patient with its opening in register with the artificial anus, an inflatable padding, and inflation means, the bag being **characterized in that** the padding is constituted by a cushion (3) that is disposed between the front and back walls (11, 12) of the receptacle and that extends from the periphery (14) of said receptacle to an outline of the cushion situated at a sufficient distance from the opening (13) to ensure that the swelling formed by the inflated cushion does not transmit pressure to the artificial anus when the bag is put in place.

2. A bag according to claim 1, **characterized in that** the inflation means also enable deflation.

3. A bag according to claim 2, **characterized in that** the inflation means comprise a non-return valve (4) having at least a portion inside the cushion (3).

4. A bag according to claim 3, **characterized in that** the valve (4) also has an outside portion adapted to receive an end piece, enabling a user to blow air therein for the purpose of inflating the cushion (3).

5. A bag according to claim 4, **characterized in that** the valve (4) is formed by two walls (41, 42) that are flexible and impermeable to gas, that face each other, that are welded together laterally, and that are provided with means for stiffening the walls (41, 42) of the valve (4) transversely so that the walls are pressed against each other in airtight manner under the effect of the pressure that exists inside the cushion (3).

6. A bag according to claim 5, **characterized in that** the means for stiffening the walls (41, 42) of the valve (4) transversely are constituted by a transverse weld (14) between the walls (41, 42) of the valve, a passage (43) being formed through the transverse weld (14) to provide communication between the cushion (3) and the outside.

7. A bag according to claim 4, **characterized in that** the valve (4) is formed by two rigid pieces that terminate at the end of the valve inside the cushion (3) in sufficiently tapering manner to enable them to be pressed against each other in airtight manner by the pressure that exists inside the cushion (3).

8. A bag according to claim 7, the front and back walls (11, 12) being welded together along a peripheral weld (14) which defines the periphery of the receptacle (1), the bag being **characterized in that** the cushion (3) is constituted by two walls (31, 32) that are flexible and impermeable to gas, that face each other, and that are welded together at the peripheral weld (14) and also along an inside weld (34) following the outline of the cushion (3).

9. A bag according to claim 8, **characterized in that** a third wall (33) that is flexible and impermeable to gas has both edges engaged in the peripheral weld (14) of the receptacle (1) so as to form a bellows making a larger inflation volume possible.

10. A bag according to claim 7, the front and back walls (11, 12) being welded together along a peripheral weld (14) which defines the periphery of the receptacle (1), the bag being **characterized in that** the cushion (3) is constituted by a wall (31) that is flexible and impermeable to gas, and that is welded to the back wall (12) of the receptacle (1) at the peripheral weld (14) and also along an internal weld (34) following the outline of the cushion (3).

11. A bag according to claim 10, **characterized in that** the wall (31) constifuting the cushion (3) is an intermediate wall of the receptacle which is welded to the front and back walls (11, 12) along the entire peripheral weld (14), and also to the back wall (12) along an additional weld (15) surrounding the inlet opening (13) for excreta so that the excreta is stored solely between the front wall (11) and the intermediate wall.

12. A bag according to claim 11, **characterized in that** the intermediate wall includes at least one fold (36) adapted to form a bellows in the cushion (3) to make a greater inflation volume possible.

13. A bag according to any one of claims 7 to 12, in which the receptacle (1) once placed on the patient has a top portion and a bottom portion, the bag being **characterized in that** the cushion (3) extends in at least the top portion of the receptacle (1).

14. A bag according to claim 13, **characterized in that** a vent filter is disposed on the front wall (11) of the receptacle (1) at the same height as the cushion (3).
